# EUROPEAN PATENT APPLICATION

(11) **EP 4 635 520 A1**
(43) Date of publication of application: **22.10.2025**
(21) Application number: 23903661.9
(22) Date of filing: 31.07.2023
(51) Int. Cl.: A61K 48/00, A61K 31/7088, A61K 9/00, A61P 19/02, A61L 27/54, A61L 27/36

(54) **COMPOSITION FOR INJECTION INTO JOINT CAVITY OF DOGS OR CATS COMPRISING SODIUM POLYNUCLEOTIDE**

(30) Priority: 15.12.2022 KR 20220176310
(71) Applicant: Pluto Inc., Seongnam-si Gyeonggi-do 13453 (KR)
(72) Inventor: JEON, Hong Ryeol, Suwon-si, Gyeonggi-do 16543 (KR); KWON, Dow, Suwon-si, Gyeonggi-do 16488 (KR); LEE, Sun Ahe, Suwon-si, Gyeonggi-do 16434 (KR); LEE, Bong Sang, Uiwang-si, Gyeonggi-do 16022 (KR); KIL, Myeongcheol, Seongnam-si, Gyeonggi-do 13504 (KR)
(74) Representative: Høiberg P/S
(86) International application number: PCT/KR2023/011171
(87) International publication number: WO 2024/128457

(57) **Abstract**

The present invention relates to a composition for injection into the joint cavity of dogs or cats, which is used to treat or ameliorate arthritis by injection into the joint cavity of dogs or cats, or to reduce mechanical friction in the joint region through physical restoration. The present invention also relates to a method for administering a composition for injection into the joint cavity of dogs or cats, the composition being used to treat or ameliorate arthritis by injection into the joint cavity of dogs or cats, or to reduce mechanical friction in the joint region through physical restoration. When the composition of the present invention is administered, the dose of sodium polynucleotide is 2-3 mg, preferably 2.5-3 mg, per body weight (kg) of a dog or a cat.

## Description

### [Technical Field]

The present invention relates to a composition for injection into the joint cavity of a dog or cat, comprising sodium polynucleotide. The present invention also relates to a method for administering the composition for injection into the joint cavity of a dog or cat, comprising sodium polynucleotide. The composition and method of the present invention are useful for treating or ameliorating arthritis in a dog or cat, and are also useful for reducing mechanical friction in the joint region through physical restoration.

### [Background Art]

Among arthritis diseases, degenerative arthritis is the most common, also called degenerative joint disease or osteoarthritis. This degenerative arthritis is a disease in which the cartilage protecting the joint is gradually damaged or degeneratively changed, causing damage to the bones and ligaments that make up the joint, causing inflammation in the synovial membrane around the joint, causing pain and deformation. Degenerative arthritis progresses through the process in which cartilage tissue in joints such as the knees, ankles, fingers, elbows, and hip joints gradually degenerates and is destroyed due to old age, obesity, joint trauma, hip dysplasia, a history of arthritis, genetic factors, etc.

This degenerative arthritis is also gradually increasing in companion dogs or cats. It is one of the diseases of old age that mainly occurs in older dogs or cats. Symptoms vary, but they mainly show a reluctance to move, show abnormal gait, and lift their legs or, in severe cases, crouch down when walking or exercising.

General drug treatment is helpful in ameliorating arthritis in companion dogs or cats. For example, non-steroidal anti-inflammatory drugs have been used in various ways recently to relieve pain and prevent and treat inflammation. However, these non-steroidal anti-inflammatory drugs can cause bloody vomiting, bloody stool, anemia, etc. due to peptic ulcers, and there is also a risk of renal failure.

Therefore, a drug that has few side effects when administered to companion dogs or cats and is effective in ameliorating arthritis is needed.

### [Disclosure]

### [Technical Problem]

Therefore, the problem that the present invention seeks to solve is to provide a preparation that has fewer side effects and is effective in ameliorating arthritis when administered to dogs or cats, especially dogs.

Another problem that the present invention seeks to solve is to provide a method for administering sodium polynucleotide that has fewer side effects and is effective in ameliorating arthritis when administered to dogs or cats, especially dogs.

### [Technical Solution]

In order to solve the above problem, one embodiment of the present invention provides a composition for injecting sodium polynucleotide into the joint cavity of a dog or cat, preferably a dog, which is used to treat or ameliorate arthritis of a dog or cat, preferably a dog, or reducing mechanical friction of a joint area through physical restoration, wherein the sodium polynucleotide is administered in a dosage of 2-3 mg per kg of the body weight of the dog or cat.

Another embodiment of the present invention provides a method for administrating a composition for treating or ameliorating arthritis of a dog or cat, preferably a dog, or reducing mechanical friction of a joint area through physical restoration by injecting sodium polynucleotide into the joint cavity of a dog or cat, preferably a dog, wherein the sodium polynucleotide is administered in a dosage of 2-3 mg per kg of the body weight of the dog or cat.

In a preferred embodiment of the present invention, the composition and the administration method of the present invention are administered so that 2.5-3 mg of sodium polynucleotide is administered per kg of body weight of the dog or cat. In a more preferred embodiment of the present invention, the composition and the administration method of the present invention are administered so that 2.5 mg of sodium polynucleotide is administered per kg of body weight of the dog or cat.

In a preferred embodiment of the present invention, the composition of the present invention is a liquid composition having a content of 20 mg/ml of sodium polynucleotide. In another preferred embodiment of the present invention, the administration method of the present invention administers a liquid composition having a content of 20 mg/ml of sodium polynucleotide.

In a preferred embodiment of the present invention, the liquid composition having a content of 20 mg/ml of sodium polynucleotide among the compositions and the administration methods is administered in a dose of 0.1-0.15 ml per kg of body weight of the dog or cat, more preferably in a dose of 0.125-0.15 ml. In a more preferred embodiment of the present invention, the liquid composition is administered in a dosage of 0.125 ml per kg of body weight of the dog or cat.

The composition of the present invention is administered into the joint cavity of a dog or cat, and is preferably administered at said dose of 2-3 mg of sodium polynucleotide per kg of body weight (more preferably 2.5-3 mg per kg of body weight), and particularly, considering the administration volume, it is preferably injected into the joint cavity as a liquid composition of 20 mg/ml to achieve these doses.

The sodium polynucleotide according to the present invention is a transparent liquid substance with viscosity, which is a DNA fragment extracted from the testis of salmonid fish. For example, such sodium polynucleotide can be prepared by the method described in Korean Patent No. 10-0986603. In one embodiment of the present invention, the average molecular formula of the deoxyribonucleotide constituting the DNA fragment mixture of the sodium polynucleotide is C_{9.83}H_{12.33}N_{3.72}O_{6.01}P·Na. In one embodiment of the present invention, the molecular weight of sodium polynucleotide exhibits a maximum distribution of sodium polynucleotide at 250-1,000 base pairs (bp) when tested using an electrophoresis method, exhibits an absorption maximum at 260±2 nm when measuring absorbance, and the ratio of absorbance at 260 nm and 280 nm is 1.6 to 2.0. In addition, the pH of a 1 wt% aqueous solution of sodium polynucleotide according to the present invention is 6.5-7.5.

In one embodiment of the present invention, the sodium polynucleotide comprised in the composition of the present invention has a content of 20 mg/ml, and the composition of the present invention has a viscosity of 100-300 Pas when measured according to the viscosity measurement method of the General Test Method of the Korean Pharmacopoeia, and an elasticity of 50-250 Pa when measured under the conditions of Geometry: PU20, Temperature: 25°C, and Frequency: 0.1Hz.

In one embodiment of the present invention, Conjuran^{™}, a liquid composition having a sodium polynucleotide content of 20 mg/ml, may be used as the composition of the present invention.

In another embodiment of the present invention, the sodium polynucleotide composition may be prepared as described in Korean Patent Publication No. 10-2017-0100236, and the contents described in Korean Patent Publication No. 10-2017-0100236 are incorporated herein by reference in their entirety.

In one embodiment of the present invention, the composition of the present invention is administered to the joint cavity 2-4 times in total at 2-4 week intervals. In a preferred embodiment of the present invention, the composition of the present invention is administered to the joint cavity 2 times in total at 2 week intervals.

### [Advantageous Effects]

The present invention provides a composition for injection into the joint cavity of dogs or cats, preferably dogs, which is used to treat or ameliorate arthritis, or to reduce mechanical friction in the joint region through physical restoration by being injected into the joint cavity. In particular, the composition of the present invention is administered such that the dosage of sodium polynucleotide is 2-3 mg per kg of body weight of the dog or cat, preferably 2.5-3 mg, more preferably 2.5 mg.

### [Brief Description of Drawings]

Figure 1 is a photograph of a 20 mg/ml sodium polynucleotide preparation administered into the joint cavity of a companion dog showing arthritis symptoms.
Figures 2 and 3 are the results of the behavioral evaluation, and the results of the behavior index and the standing index, respectively. The values of the behavior index and the standing index for the knees of the affected dogs in the saline and sodium polynucleotide administration groups during the 30-day study period.
Figures 4 and 5 are the results of the pain relief evaluation, and the results of the evaluation of 'the degree of pain complaints upon palpation and movement' and 'the degree of weight bearing on the affected hind limb', respectively. The values of the behavior index and the standing index for the knees of the affected dogs in the saline and sodium polynucleotide administration groups during the 30-day study period.
Figure 6 shows the results of evaluating the effect of test substance on the concentration of IL-1β in serum 48 weeks after the first intra-articular injection in the serum of dogs with osteoarthritis.

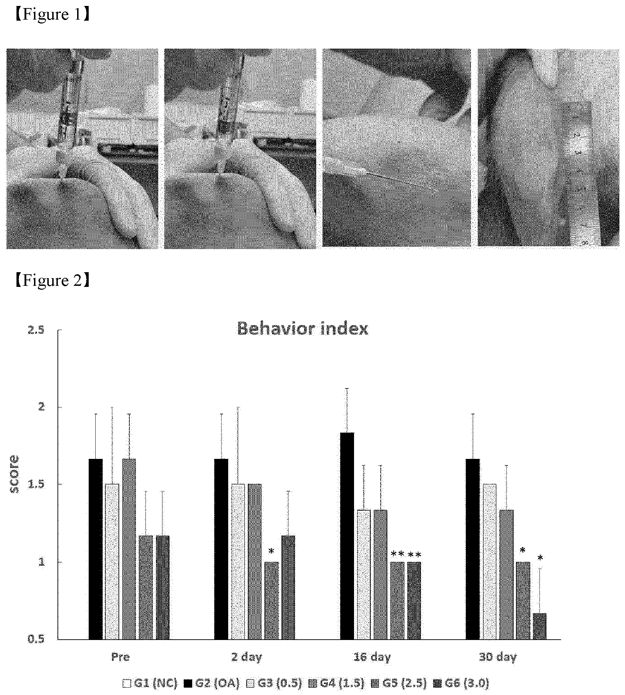

In Figures 2 to 6, the bar graphs represent G1 (NC) (no bar), G2 (OA), G3 (0.5), G4 (1.5), G5 (2.5), and G6 (3.0) from the left in order. In Figures 2 to 6, * and ** represent data that show statistically significant differences (p<0.05, p<0.01, respectively) compared to the OA group at a specific time point, and the meanings of each abbreviation are as follows:
G1 (NC): normal group, G2 (OA): anterior cruciate ligament transection with medial meniscectomy (ACLT/MMx)-induced osteoarthritis group, G3 (0.5): ACLT/MMx operated and treated with Sodium Polynucleotide 0.5 mg/kg, G4 (1.5): ACLT/MMx operated and treated with Sodium Polynucleotide 1.5 mg/kg, G5 (2.5): ACLT/MMx operated and treated with Sodium Polynucleotide 2.5 mg/kg, G6 (3.0): ACLT/MMx operated and treated with Sodium Polynucleotide 3.0 mg/kg, WILL: Willingness to allow the clinician to lift the limb contralateral to the affected limb.

### [Mode for Invention]

Hereinafter, in order to help understand the present invention, examples and the like will be described in detail. However, the examples according to the present invention may be modified in various different forms, and the scope of the present invention should not be construed as being limited to the following examples. The examples of the present invention are provided to more completely explain the present invention to a person having average knowledge in the field to which the present invention pertains.

In the preliminary experiment, when a lower dose than 2.5 mg per kg of body weight was administered, especially when a dose of less than 2.0 mg per kg of body weight was administered, satisfactory effects were not observed. In addition, considering the intra-articular volume of companion dogs, it was most appropriate to use a 20 mg/ml sodium polynucleotide preparation, that is, to administer approximately 0.125 ml per kg of body weight so that the dosage was 2.5 mg/kg.

Therefore, in the following experiments, 10 companion dogs of various species (body weight: 3 kg to 10 kg) showing arthritis symptoms were injected intra-articularly with a 20 mg/ml polynucleotide sodium preparation (Conjuran^{™}) so that 2.5 mg as sodium polynucleotide per kg of body weight (approximately 0.125 ml per kg of body weight) was administered (see figure 1).

### Experimental Example 1. Evaluation of Pain Complaints During Palpation and Mobilization

Pain relief was evaluated by the degree of pain complaints during palpation and mobilization. The degree of pain was scored according to the classification of the grading table specified in Table 1 below for each evaluation index. Pain evaluation was conducted before and after administration.

**[Table 1]**

| Score | Pain at palpation/mobilization | Behavioral supplements related to walking posture and pain, psychological evaluation |
|---|---|---|
| 0 | No pain elicited on palpation/mobilization of the affected joint | Normal group |
| 1 | Mild pain elicited on palpation/mobilization of the affected joint, e.g., turns the head in recognition | When limping while walking on four limbs, the change in trunk height is low or the speed of leg switching is normal |
| 2 | Moderate pain elicited on palpation/mobilization of the affected joint, e.g., pulls the limb away | When walking, the tendency to move on three legs is about 30% or when limping while walking on four limbs, the change in trunk height is large or the speed of leg switching is high |
| 3 | Severe pain elicited on palpation/mobilization of the affected joint, e.g., vocalizes or becomes aggressive | Walking on three legs |
| 4 | Severe pain elicited on palpation/mobilization of the affected joint, e.g., not allow examiner to palpate/mobilize the joint | Unable to walk even on three legs and lack of vitality, clear warning when approaching |

The evaluation results, including the degree of pain complaints during palpation and mobilization, are summarized in Table 2 below.

**[Table 2]**

| | Before administration | After administration |
|---|---|---|
| Individual 1 | 3 points | 1 point |
| Individual 2 | 4 points | 2 points |
| Individual 3 | 2 points | 0 point |
| Individual 4 | 3 points | 1 point |
| Individual 5 | 3 points | 1 point |
| Individual 6 | 3 points | 2 points |
| Individual 7 | 3 points | 2 points |
| Individual 8 | 2 points | 1 point |
| Individual 9 | 3 points | 2 points |
| Individual 10 | 3 points | 2 points |

The test substance had physical properties that made it easy to inject into the joint cavity, and the administered individuals (dogs) 1 to 10 showed a decrease in pain.

### Experimental Example 2. Standing Evaluation

The standing scale was evaluated according to the classification of the rating scale specified in Table 3 below. That is, scoring was performed on behavioral status. During the behavioral evaluation, the animals were allowed to walk freely while allowing an adaptation time of 5 to 10 minutes. The corresponding standing index was assigned a score while walking.

**[Table 3]**

| Score | Standing index | Basic posture and hind leg load evaluation |
|---|---|---|
| 0 | Normal stance (Standing posture with full weight bearing) | Normal group |
| 1 | Slightly abnormal stance (partial weight-bearing of the limb, but the paw remains firmly in contact with floor) | - In a standing state with all feet firmly on the floor, when the hind legs on both sides are loaded, the resistance is almost the same as the left leg and the state of contact is maintained |
| 2 | Markedly abnormal stance (partial weight-bearing of the limb, with minimal contact between the paw and the floor) | - In a standing position with all feet firmly on the floor, the left leg is separated with low resistance when the left and right hind legs are loaded |
| 3 | Abnormal standing position with partial weight bearing condition (using 3 legs) | 3-leg standing and repeated contact and separation from the floor |
| 4 | Do not try to stand | Unable to stand |

The evaluation results are summarized in Table 4 below.

**[Table 4]**

| | Before administration | After administration |
|---|---|---|
| Individual 1 | 3 points | 2 points |
| Individual 2 | 4 points | 2 points |
| Individual 3 | 2 points | 0 point |
| Individual 4 | 3 points | 1 point |
| Individual 5 | 3 points | 1 point |
| Individual 6 | 3 points | 2 points |
| Individual 7 | 3 points | 2 points |
| Individual 8 | 2 points | 1 point |
| Individual 9 | 3 points | 2 points |
| Individual 10 | 3 points | 2 points |

As shown in Table 4 above, when the test substance was injected into the joint cavity, the overall foot strength was increased and the standing test showed improvement.

### Experimental Example 3. Evaluation of the effect according to the dose

As follows, osteoarthritis was induced in beagle dogs (18 males, 2.5-5 years old, 12-16 kg) by anterior cruciate ligament resection and medial meniscectomy for about 3 months, and the effect according to the dose of the composition of the present invention was compared and evaluated.

The beagle dog species used was *Canis familiaris* (Woojungbio., Co. Ltd.). The beagle dog selected for this study can be used as a surgically induced osteoarthritis model, and it is an animal species that is easy to evaluate the analgesic and behavioral improvement effects of test substances, and since there is accumulated prior research data, it can be easily interpreted and evaluated the test results. After approximately 2 weeks of acclimatization and drug withdrawal prior to the test, healthy animals suitable for conducting the experiment were used. On the day of surgery and the day of application of the test substance, the individual was anesthetized under a 16-hour fasting, and after recovery from anesthesia, approximately 300 g/day/head of solid food was fed to the feeder and allowed to eat freely. In addition, 300 g/day/head was fed once a day in the afternoon at a certain time during the test period and allowed to eat freely.

Thereafter, the composition of the present invention was administered according to Table 5 below. Intra-articular administration was performed for all administration groups. Before administration of the test substance, the animals were anesthetized under 16-hour fasting and placed in the supine position. The administration composition was administered using a 3cc syringe (with a 23 G needle attached). With the right knee bent, the injection was made into the knee joint cavity via the patellar tendon according to the administration volume of each group. The test substance was administered twice in total (day 0, day 14) at 2-week intervals after arthritis was induced and the groups were separated.

**[Table 5]**

| Groups | | OA Surgery | Dosage (mg/kg) | Volume (mL/kg) | Route of administration | Number (N) |
|---|---|---|---|---|---|---|
| G1 | NC | - | - | - | - | 3 |
| G2 | OA | ACLT + | - | - | - | 3 |
| G3 | Sodium polynucleotide 20 mg/ml preparation | MMx | 0.5 | 0.025 | I.A | 3 |
| G4 | Sodium polynucleotide 20 mg/ml preparation | | 1.5 | 0.075 | I.A | 3 |
| G5 | Sodium polynucleotide 20 mg/ml preparation | | 2.5 | 0.125 | I.A | 3 |
| G6 | Sodium polynucleotide 20 mg/ml preparation | | 3.0 | 0.150 | I.A | 3 |

| | | | | | | |
|---|---|---|---|---|---|---|
| OA: osteoarthritis, ACLT: anterior cruciate ligament transection, MMx: medial meniscectomy, I.A: intra articular | | | | | | |

The test group consisted of a total of 6 groups (n=3), the negative control group was group G1, the induced group was group G2, and the test groups were groups G3-G6. In groups G2-G6, osteoarthritis was induced through an anterior cruciate ligament section and medial meniscectomy by incision of the right knee joint. On the day of test substance administration, group G1 did not receive any separate treatment, while group G2 received the same amount of 0.9% saline into the joint cavity. The first day of test substance administration was designated as day 0.

Osteoarthritis was induced as follows. Fifteen animals to be induced with osteoarthritis were injected subcutaneously with atropine sulfate (0.03 mg/kg) and prednisolone as internal medicine before the surgical procedure under a 16-hour fast. Cefazolin (20 mg/kg) and meloxicam (0.2 mg/kg) were injected intramuscularly and subcutaneously as antibiotics and anti-inflammatory analgesics before the surgery, respectively. Anesthesia was maintained by intravenous injection of Zoletil50 (10 mg/kg) and xylazine (0.2 mg/kg). The hair on the right knee joint and the thigh around the knee joint of each animal was removed using a hair remover and disinfected with betadine. To ensure smooth hemostasis and infiltration anesthesia during surgery, 2 mL of 2% lidocaine (1:10,000 epinephrine) as a local anesthetic was injected around the knee joint, and then an approximately 5 cm longitudinal incision was made centered on the tibial tubercle from the medial parapatellar approach. After incising the exposed joint cavity, the patella was positioned laterally, and the knee was flexed to expose the anterior cruciate ligament and meniscus under the surgical field. The middle part of the anterior cruciate ligament was completely cut with a surgical blade (No. 15), and the medial meniscus was grasped and pulled with forceps, and the meniscus ligament was cut from its surrounding attachments using surgical scissors to completely remove the medial meniscus. Afterwards, it was confirmed that the ligament's support for the tibialis was loosened through the anterior drawer's test, and the joint cavity and subcutaneous tissue were each sutured using absorbable sutures, and the skin was sutured with non-absorbable sutures. For postoperative pain control, meloxicam (0.2 mg/kg) was injected subcutaneously for 5 days, and concurrently, the surgical site was disinfected with betadine for about 7 days to prevent infection, and cefazolin was injected intramuscularly at a dose of 20 mg/kg twice a day for 7 days. All animals were allowed to move freely in individual cages for about 12 weeks to aid recovery without external fixation. All animals underwent a stabilization period of approximately 4 weeks to allow the skin over the surgical incision site to stabilize. Afterwards, to induce osteoarthritis, the animals were positioned supine on a table by a correction agent for approximately 3-5 minutes once daily for 8 weeks, while another experimenter repeatedly performed flexion and relaxation exercises on the operated right knee joint. The animals were then returned to their cages and allowed to move freely without separate external fixation.

All experimental results obtained in the experiment are expressed as the mean ± standard deviation and were tested using SPSS (version 20, IBM SPSS Statistics, USA). In the evaluation results, comparisons between the normal group (group G1) and the induced group (group G2) and comparisons between the induced group (group G2) and the test group were tested at the p<0.05 level using the student's T-test method (significance level: two-sided 5%, 1%, and 0.1%).

### Behavioral evaluation (evaluation of behavior index and standing index)

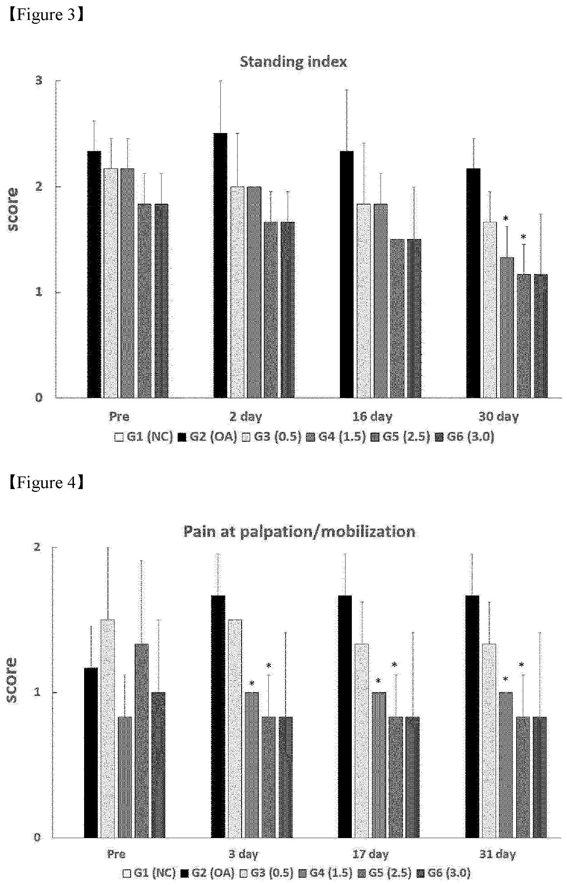

The behavioral evaluation consisted of a behavior index and a standing index, and the behavior index and standing index of the G1 to G6 test groups were evaluated according to Table 6 below or Table 3 above, respectively. When evaluating the behavior, the evaluation animals were placed in an empty space of about 8 pyeong without people, and allowed to walk freely for about 5 to 10 minutes of adaptation time. An observer gave the corresponding score for each behavioral evaluation index through an open window that allowed the room to be seen while walking. The behavioral evaluation was conducted a total of 4 times, and the evaluation was conducted 2 days after the end of the test substance administration on the first (day 0) and second (day 14) administration days, and 2 weeks after the second administration. The results are shown in Figures 2 and 3, respectively.

**[Table 6]**

| Score | Behavior index | Behavioral evaluation under pain due to surgery for disease induction | |
|---|---|---|---|
| 0 | Indifferent | Normal group | |
| 1 | Friendly | High | In a state of pain due to surgery, the level of intimacy with people (vitality, tail wagging, approachability, facial expression, alertness, gait, etc.) is comprehensively judged and divided into high, medium, and low. |
| 2 | Nervous, submissive behavior | Medium | |
| 3 | Very nervous, tries to move away | Low | |
| 4 | Aggressive | A state of extreme alertness and inaccessibility | |

As a result of the behavior index evaluation, the G5 (2.5) group among the test groups showed the best result with 1.00 after 2 days from the 1^{st} administration. When evaluated on the 16^{th} day (2 days after the 2^{nd} administration), the G5 (2.5) group and G6 (3.0) group showed an average of 1.00 and 1.00, respectively, and when evaluated on the 30^{th} day (16 days after the 2^{nd} administration), the G5 (2.5) group and G6 (3.0) group showed an average of 1.00 and 0.67, respectively. When compared to the OA group, which showed 1.67, 1.83, and 1.67 at each evaluation time point, a significant decrease was confirmed in these high-dose (G5 (2.5) group, G6 (3.0) group) test groups (p<0.05, figure 2).

There was no test group that showed a significant difference in the standing index evaluation results compared to the OA group at the 2^{nd} and 16^{th} day evaluations from the start of the first administration. At the final evaluation on the 30^{th} day, the average values were 1.33, 1.17, and 1.17 in the G4 (1.5) group, G5 (2.5) group, and G6 (3.0) group, respectively, showing a significant decrease in the G4 (1.5) and G5 (2.5) groups compared to the OA group, which showed 2.17 (p<0.05, figure 3).

### Pain relief evaluation

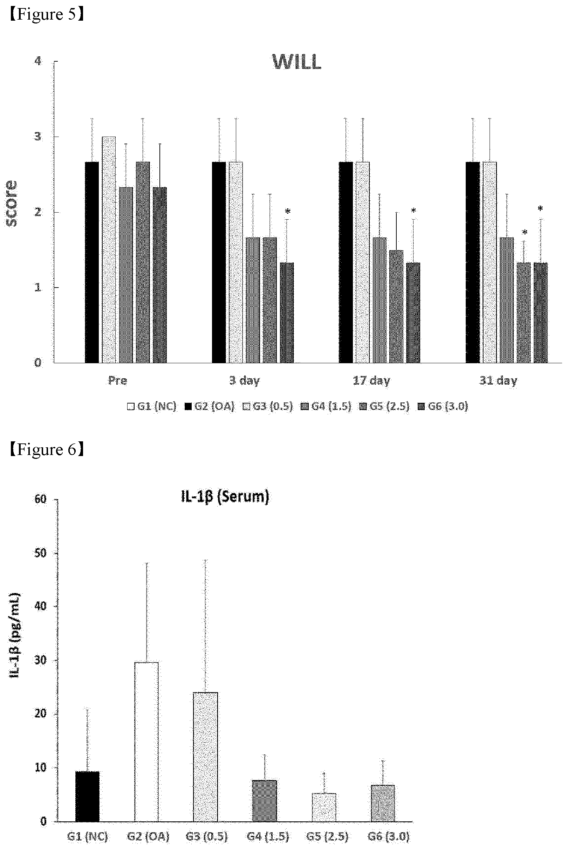

Pain relief evaluation consisted of the degree of pain complaints during palpation and mobilization and the degree of weight bearing on the affected hind limb. The degree of pain was scored according to the grading table classification specified in Table 1 above and Table 7 below for each evaluation index. Pain evaluation was conducted a total of 4 times, and the evaluation time was conducted on the day after the 1^{st} (day 0) and 2^{nd} (day 14) administration and 2 weeks after the 2^{nd} administration. All pain evaluations were conducted on the day after the behavioral evaluation. The results are shown in figures 4 and 5.

**[Table 7]**

| Score | Willingness to allow the clinician to lift the limb contralateral to the affected limb | Power and resistance applied when supporting the affected area alone | |
|---|---|---|---|
| 0 | Readily accepts contralateral limb elevation, bears full weight on the affected limb for more than 30 seconds | Corresponds to the normal group | |
| 1 | Offers mild resistance to contralateral limb elevation, bears full weight on the affected limb for more than 30 seconds | Low | Classified by the level of power and resistance applied to the left leg and foreleg when supporting the affected area alone (basic balance and posture between individuals, etc. - consider the optimal tilt without losing balance in order to support the affected area as much as possible) |
| 2 | Offers moderate resistance to contralateral limb elevation and replaces it in less than 30 seconds | Med ium | |
| 3 | Offers strong resistance to elevation of contralateral limb and replaces it in less than 10 seconds | High | |
| 4 | Refuses to raise contralateral limb | Rejecting support of the affected area alone | |

The results of the pain at palpation/mobilization evaluation showed that the average values were 1.00 and 0.83, respectively, in the G4 (1.5) group and the G5 (2.5) group among the test groups at all evaluation time points (days 3, 17, and 31), showing a significant decrease compared to the OA group, which showed the same value of 2.50 at each evaluation time point (p<0.05, figure 4).

As a result of evaluating the Willingness to Allow the Clinician to Lift the Limb Contralateral to the Affected Limb (WILL), the high-dose group G5 (2.5) among the test groups showed 1.67, 1.50, and 1.33 at all evaluation time points (3^{rd}, 17^{th}, and 31^{st} days), which was a significant decrease compared to the OA group, which showed 2.67 at the 31^{st} day. In the highest-dose group, the average value was commonly confirmed to be 1.33, which showed a significant decrease compared to the OA group, which showed 2.67 at all 3, 17, and 31 days (p<0.05, figure 5).

### Analysis of cytokines related to inflammation in blood

According to previous studies, osteoarthritis is a non-inflammatory arthritis, but as cartilage destruction progresses, changes related to inflammation occur, and the articular capsule shows a mild to moderate inflammatory response, which is partially due to the inflammatory response caused by the destroyed cartilage fragments in the synovial fluid. Once an inflammatory response occurs in the synovium, synoviocytes produce cartilage-destructive enzymes such as matrix metalloproteinases, interleukin-1 (IL-1), interleukin-6 (IL-6), and tumor necrosis factor-alpha (TNF-alpha), which stimulates chondrocytes to produce more destructive enzymes. In addition, potent inflammatory mediators are released, and IL-1, which is the most potent in destroying cartilage, mediates the inflammatory response by causing synovial cells to produce prostaglandin E2, downregulating the synthesis of extracellular matrix, and upregulating matrix metalloproteinase (MMP) through nitric oxide (NO) production in chondrocytes. Therefore, we aimed to evaluate the degree of treatment of canine arthritis through the blood level of IL-1β.

Blood collection was performed on all subjects 2 weeks after the second administration of the test substance under a 16-hour fast. 10 ml of blood was collected from each subject and divided into two SST (BD Vacutainer SST tube, 5 ml) tubes. The blood was centrifuged at 4,000 rpm for 10 minutes at 4°C. The separated supernatant was divided into 5 sets of 1 ml each on 1.5 ml EP tubes and stored in a -80°C deep freezer until analysis.

IL-1β, a representative inflammatory cytokine in serum, was measured using an ELISA kit (CUSABIO, CSB-E13836c) and analyzed using an ELISA reader (SpectraMax M2, Molecular Devices, USA). The results are shown in figure 6.

As shown in figure 6, the serum average value (pg/ml) of the IL-1β quantitative results was confirmed to be NC group (G1, 9.27), OA group (G2, 29.56), G3 (0.5) group (23.96), G4 (1.5) group (7.63), G5 (2.5) group (5.22), and G6 (3.0) group (6.74). In the serum IL-1β index results, all test groups showed lower levels than OA, and among them, G4 (1.5), G5 (2.5), and G6 (3.0) groups showed lower levels than the NC group. That is, it was confirmed that the composition of the present invention showed a result of lowering the increase in the level of inflammatory cytokine (IL-1β) overall, thereby showing a positive effect on pain and behavior improvement.

### Conclusion

In summary, in the surgically induced beagle osteoarthritis model under the test conditions, the sodium polynucleotide 20 mg/ml preparation as an injection as a test substance showed significantly lower scores in the behavioral evaluation index (behavioral index and standing index) and pain evaluation index (pain level upon palpation and mobilization and weight-bearing level of the affected hind limb) compared to the induced group after intra-articular administration, and it was confirmed that the mechanism was to suppress the increase in the level of inflammatory cytokine (IL-1β) and show behavioral improvement and analgesic effects. In particular, this effect was confirmed to be the most effective at the same level at doses of 2.5 mg/kg and 3.0 mg/kg of the sodium polynucleotide 20 mg/ml preparation.

## Claims

1. A composition for treating or ameliorating arthritis of a dog or cat or reducing mechanical friction of a joint area through physical restoration by intra-articular injection of sodium polynucleotide,
wherein the composition is **characterized in that** the sodium polynucleotide is administered in a dose of 2-3 mg per kg of body weight of the dog or cat.

2. The composition of claim 1, wherein the sodium polynucleotide is administered in a dose of 2.5-3 mg per kg of body weight of the dog or cat.

3. The composition of claim 2, wherein the sodium polynucleotide is administered in a dose of 2.5 mg per kg of body weight of the dog or cat.

4. The composition of claim 1, wherein the composition is a liquid composition having a content of sodium polynucleotide of 20 mg/ml.

5. The composition of claim 4, wherein the liquid composition is administered in a dose of 0.1-0.15 ml per kg of body weight of a dog or cat.

6. The composition of claim 5, wherein the liquid composition is administered in a dose of 0.125-0.15 ml per kg of body weight of a dog or cat.

7. The composition of claim 6, wherein the liquid composition is administered in a dosage of 0.125 ml per kg of body weight of a dog or cat.

8. The composition of any one of claims 1 to 7, wherein the composition is for intra-articular administration of a dog or cat.

9. The composition of claim 8, wherein the composition is for intra-articular administration of a dog.

10. The composition of any one of claims 1 to 7,, wherein the sodium polynucleotide comprised in the composition is a DNA fragment extracted from the testis of a salmonid.
